# EUROPEAN PATENT SPECIFICATION

(11) **EP 3 077 786 B1**
(45) Date of publication and mention of the grant of the patent: **17.03.2021**
(21) Application number: 14867654.7
(22) Date of filing: 05.12.2014
(51) Int. Cl.: G01N 1/20, G01N 33/28, G01N 1/10

(54) **METHOD FOR ANALYSIS OF A LIQUID WITH A SAMPLING UNIT FOR A LIQUID SAMPLE ADAPTED TO BE FITTED INTO A SYSTEM WITH TEMPERATURE VARIATIONS**
VERFAHREN ZUR ANALYSE EINER FLÜSSIGKEIT MIT EINER EINHEIT ZUR ENTNAHME EINER FLÜSSIGKEITSPROBE ZUM EINBAU IN EINEM SYSTEM MIT TEMPERATURSCHWANKUNGEN
PROCÉDÉ D'ANALYSE D'UN LIQUIDE AVEC UNE UNITÉ D'ÉCHANTILLONNAGE POUR UN ÉCHANTILLON DE LIQUIDE, CONÇUE POUR ÊTRE MONTÉE DANS UN SYSTÈME AYANT DES VARIATIONS DE TEMPÉRATURE

(30) Priority: 06.12.2013 SE 1351454
(43) Date of publication of application: 12.10.2016
(73) Proprietor: Scania CV AB, 151 87 Södertälje (SE)
(72) Inventor: ERIKSSON, Henrik, S-141 59 Huddinge (SE); EURENIUS, Klas, 151 46 Södertälje (SE); FORSBERG, Carina, S-152 51 Södertälje (SE); JONSSON, Anders, S-144 52 Rönninge (SE)
(74) Representative: Scania CV AB
(86) International application number: PCT/SE2014/051456
(87) International publication number: WO 2015/084249

(56) References cited:
- EP-A2- 2 405 251
- WO-A1-2004/003343
- WO-A1-2011/029450
- WO-A1-2013/002713
- WO-A2-2012/078327
- CN-U- 201 837 541
- DE-A1- 4 301 174
- FR-A1- 2 656 694
- US-A1- 2002 079 236
- US-A1- 2009 211 379

## Description

### FIELD OF TECHNOLOGY

The invention relates to a method for sampling of a liquid.

### BACKGROUND OF THE INVENTION AND PRIOR ART

Diesel-powered motor vehicles are equipped with exhaust purification devices with the objective of reducing emissions of particles and chemicals occurring in the diesel engine's exhausts. There are also different standards and statutory requirements governing allowable exhaust emissions from vehicles. Prior art exhaust purification devices are sensitive to high levels of sulphur in the fuel. A level exceeding 10 ppm of sulphur in the fuel may lead to a poor reduction of emissions in the exhaust purification device. In order to reduce this risk, and ensure that the legislative requirements are met, some vehicle manufacturers specify the maximum level of sulphur that the fuel may contain, e.g. the level of sulphur must be less than 10 ppm. However, it is difficult to determine in retrospect, and to prove, that the vehicle was fuelled with fuel with a sulphur level exceeding 10 ppm.

Also other compounds, such as phosphorous compounds, may be damaging to the vehicle and mainly the environment. Likewise, it is difficult in connection with these compounds, however, to determine in retrospect, and to prove, that the vehicle was fuelled with fuel containing harmful concentrations of unwanted chemicals.

In order to reduce emissions of particles and nitrogen oxides (NOx), exhaust purification systems are used that may comprise e.g. a diesel oxidation catalyst (DOC), particulate filters and so-called NOx reducers, e.g. EGR (Exhaust Gas Reduction) systems and selective catalytic reduction (SCR) systems in the exhaust stream from combustion engines, e.g. in vehicles. The efficiency of such exhaust purification systems, and especially diesel oxidation catalysts, decreases at the occurrence of compounds containing e.g. sulphur. Such sulphur-containing compounds (e.g. mercaptans, thiols, thiophenes, thioethers, thioesters, disulfides), and especially sulphur-containing aromatic compounds, "poison" or react with the diesel oxidation catalyst and/or other components of the exhaust purification system, and these parts of the system therefore become less efficient, leading to corrosion problems in the engine and increased exhaust emissions. The diesel oxidation catalyst is sensitive to high levels of sulphur, and may therefore have a shortened life at excessively high sulphur levels in the fuel. It is therefore important to be able to analyse whether the vehicle has been fuelled with fuel containing too high a sulphur level.

In prior art technology, different methods have been tried to analyse sulphur content.

For example, US-2002/0079236 shows a sensor to measure the concentration of sulphur compounds in a liquid. The sensor comprises two electrodes, one active electrode which is in contact with the liquid to be measured, and a reference electrode which is isolated from the liquid. A voltage is generated between the electrodes, depending on the concentration of sulphur compounds in the liquid, and the concentration may therefore be determined.

US-2009/0317299 relates to an optical sensor to determine the sulphur level in a fuel. This is done by illuminating the fuel with a suitable wavelength spectrum, detecting the reflected light and then analysing this, in order to obtain a detection signal which specifies the sulphur level.

Both these prior art sensors are deemed to be active, since they require some form of power supply in connection with the detection or when the signal processing is carried out. The measurements described in both these published patent applications provide a direct measurement result, i.e. a measurement value that reflects the current sulphur level.

A proposed alternative to an electrically powered sensor is described in WO 2013/002713 A1, which shows an indication unit with a number of capsules or layers, which absorb sulphur when in contact with the fuel. The capsules each contain liquid with different sulphur levels, and the layers have different sulphur uptake capacities. At the analysis of the sulphur level in the capsules/layers, it is determined whether the level has increased and exceeds the original levels. If so, this is an indication that sulphur from the fuel has been supplied and that the sulphur level in the fuel has exceeded the predetermined level of the relevant capsule/layer.

WO2013002713 discloses a device and method for sampling a fluid. A semipermeable membrane is used to allow liquid to be sampled into a sampling unit.

Despite prior art solutions, there is a need for easily being able to detect and/or analyse the occurrence and level of sulphur contaminants and other environmentally hazardous compounds in the fuel. There is also a need to gather information about the fuel's maximum level of a chemical, e.g. sulphur, that the combustion engine has been subjected to, since a fuel with too high a sulphur level increases the risk of the catalyst's function deteriorating, and the emission requirements for the exhausts may therefore not be met. If the catalyst's function deteriorates so that the emission requirements are not complied with, and if the vehicle has been fuelled with a fuel with a sulphur level exceeding 10 ppm, this may, in the worst case, entail that the vehicle manufacturer must recall and fix a large number of vehicles, which may be very costly. If, on the other hand, it can be proven that the instructions have not been followed, in that fuel with more than e.g. 10 ppm has been used, such a campaign will not be necessary.

### SUMMARY OF THE INVENTION

Despite prior art solutions, there is a need to further develop sampling of a fluid, especially of a fuel, continuously or during a period of time, which gives an indication, in a simple way, of the level of a substance accumulated over a time period. If the sampling unit is used in a vehicle, it is possible to analyse whether the vehicle has been fuelled with fuel exceeding predetermined levels for different chemicals. There is also a need for a sampling unit which is passive and does not require any maintenance and therefore has a low cost.

The objective of the present invention is thus to provide a method for analysis of a liquid with a sampling unit which, in a simple way, is adapted to collect a liquid sample, especially a fuel sample, for further analysis. The sample may then provide an indication as to whether the vehicle has been fuelled with fuel having chemical levels exceeding approved levels for the fuel, for example the level of sulphur.

These objectives are achieved with a method for analysis of a liquid with a sampling unit defined in claim 1.

The present invention uses a sampling unit, which is equipped for a liquid sample, preferably for a fuel intended for a combustion engine. The sampling unit is adapted to be fitted in a system with pressure variations, which system contains or transports liquid. The sampling unit comprises a wall section partly surrounding a cavity is filled with a liquid, an opening equipped with a blocking element, which is adapted to be in contact with the opening and the liquid in the system. The blocking element prevents the liquid's flow into or out of the cavity at a substantially stable pressure, but allows the liquid to flow into the cavity when the pressure in the system increases from a resting pressure to an operating pressure, and out of the cavity when the pressure in the system drops back from the operating pressure to the resting pressure. The blocking element prevents the liquid's continuous inflow and outflow, e.g. flow in situations where no major pressure variations occur in the system. A substantially stable pressure means that the pressure variations in the system are minor, and smaller than the variations between the resting pressure and the operating pressure. Such small variations may be caused by e.g. variations in atmospheric pressure or disruptions during operation.

With such a sampling unit it is possible to collect a liquid sample from a system during a time period and to subsequently determine the level of a certain substance that the liquid has contained, as and when needed. The time period may be determined based on need. For example, if the DOC unit's function has deteriorated, there is a need to analyse what fuel has been fuelled. The liquid in the sampling unit contains a mixture of liquids, which have passed through the sampling unit during the time period. The mixing of liquids occurs when the liquid in a liquid-filled sampling unit is compressed at a pressure increase from a resting pressure to an operating pressure (over pressure), so that a small amount of liquid is permitted to flow into the cavity and to be mixed with the existing liquid. When the pressure in the system drops back from the operating pressure to the resting pressure, the liquid expands in the cavity of the sampling unit, and approximately the same small amount of liquid as was pressed into it, is allowed to flow out of the cavity. The amount of liquid that flows into the cavity of the sampling unit, and out of the cavity, depends on pressure variations in the system. The compression and/or expansion of the liquid is proportionate to the pressure variation and may be determined experimentally and/or by way of calculations. Since only a part of the liquid inside the sampling unit is replaced at a time, one may thus obtain an indication of the liquid's composition during the sampling period, and it is thus also possible to obtain an indication as to unlawful levels of a substance in the liquid.

When the sampling unit is used in a fuel system to collect a sample from a fuel, it is possible to obtain an indication, with the help of the invention, of e.g. the sulphur level that a fuel contained. It is therefore possible, e.g. in connection with too high sulphur levels in the fuel, to obtain an indication of the reason why an exhaust purification device has been put out of service. In the event fuel with a sulphur level above suitable levels has been used and the exhaust purification device has been put out of service, the user is informed that (s)he should use fuel with the prescribed sulphur level in the future. In the event the user did not know about the fuel's high sulphur level, the user may make demands on the fuel supplier, who must specify the correct sulphur level of the fuel.

The objectives specified above are also achieved using a system subjected to pressure variations between a resting pressure and an operating pressure, and a system comprising a hollow component, which contains or transports a liquid. The system comprises a sampling unit as described above, which is fitted inside the hollow component. Preferably, the system is a fuel system comprising a number of components, wherein at least one sampling unit as described above is fitted into at least one of the components.

The objectives specified above are also achieved using a combustion engine comprising a fuel system with a sampling unit as described above, and a vehicle comprising the combustion engine. Since there are also different standards and statutory requirements governing the permitted exhaust emissions for vehicles, it is of interest to both the vehicle manufacturer and the user of the vehicle that the exhaust purification device of the vehicle functions correctly. The sampling unit used in the invention provides both the vehicle manufacturer and the user with an indication as to whether the fuel powering the vehicle's combustion engine has had too high a level of a substance, e.g. the sulphur level.

Through the method according to the present invention, the level of a substance in the liquid may be analysed easily, e.g. the level of sulphur in a fuel.

Other features and advantages of the invention are set out in the example descriptions below.

### BRIEF DESCRIPTION OF THE DRAWINGS

Below is a description, as an example, of preferred embodiments of the invention with reference to the enclosed drawings, in which:
- Fig. 1: shows a schematic side view of a vehicle with a sampling unit,

- Fig. 2: shows an example of a coupling diagram for a fuel system,

- Figs. 3a-3d: show a schematic illustration of the sampling unit's function,

- Fig. 4: shows a cross-sectional view of a sampling unit,
- Fig. 5: shows a cross-sectional view of a sampling unit house fitted into a filter,
- Figs. 6a-6c: show a schematic functional illustration of a sampling unit,
- Figs. 7a-7c: show a schematic functional illustration of a sampling unit.

### DETAILED DESCRIPTION

The invention is described below with reference to the method as generally described above.

The sampling unit used in the present invention is intended to collect a liquid sample. Preferably, the liquid is a fuel intended for a combustion engine, but the liquid may be another liquid, which may be used within the process industry. When the sampling unit is used, it is fitted into a component in a system, which is subjected to pressure variations. The sampling unit may thus be used in a fuel system or in another system within the process industry.

The sampling unit comprises a wall section, which partly surrounds a cavity filled with a liquid, an opening through which a liquid may flow into and/or out of the cavity at pressure variations from a resting pressure to an operating pressure, and vice versa, and the opening is equipped with a blocking element, preventing the liquid's flow into or out of the cavity at a stable pressure. The blocking element is in contact with the opening and the liquid in the system, and allows the liquid to flow into the cavity through the opening when the pressure in the system increases from a resting pressure to an operating pressure, and out of the cavity through the opening when the pressure in the system drops back from the operating pressure to the resting pressure. The blocking element is adapted to be in contact with the opening and the liquid, e.g. a fuel, which is to be analysed in the system. The liquid sample may be collected continuously during a time period from the liquid in the system, with which the sampling unit is in contact. The time period is determined based on the need for analysis.

During the pressure variation, an exchange of the liquid inside the cavity occurs, because of the compression and expansion of the liquid between a resting pressure and an operating pressure. The pressure variation between a resting pressure and an operating pressure may vary greatly, and may e.g. be anywhere between approximately 6 and 2,500 bar if the sampling unit is used in the fuel system of a truck, but is not limited to such variations. The resting pressure may correspond to the atmospheric pressure, e.g. when the truck's engine is off. The operating pressure is higher than the resting pressure.

The samples are collected by the cavity first being filled with a starting liquid, which may be a "pure" liquid, e.g. fuel whose sulphur level is below 10 ppm. During the operating pressure, i.e. the over pressure, the liquid in the cavity is compressed, and thus a small amount of liquid from the surrounding system may flow into the cavity through the opening. After the pressure in the system has dropped back to the resting pressure, the liquid inside the cavity expands, and the same amount of liquid as was allowed to enter is allowed to exit through the opening. At an over pressure, liquids may mix inside the cavity.

The sampling may be continuous, and the sampling unit may be removed from the system where needed, e.g. after a time period when several cycles of over pressure and subsequent pressure drops have occurred. In this manner, there is an exchange of the liquid inside the cavity, and the sampling unit obtains a "liquid memory", which is an average composition of the liquids over the time period. The exchange time of the liquid inside the cavity varies depending on the pressure variations in the system, and the design of the sampling unit. The exchange times may be produced experimentally or by way of different calculation models. When the exchange of the liquid in the sampling unit occurs relatively slowly, it is possible to check e.g. the average sulphur level over a longer period. After the sampling unit has been removed from the system, the contents may be analysed with a suitable method.

The sampling unit is especially suitable for collecting fuel samples, especially diesel samples. The molecules in the diesel comprise mainly hydrocarbons having between 10 and 22 carbon atoms, e.g. alkanes, aromatics, naphthenes and olefins, but the fuel also contains sulphur compounds and other non-organic compounds, such as phosphorous compounds.

The sampling unit's cavity is filled with liquid and may receive the liquid sample. The cavity is partially surrounded by the wall section of the sampling unit. The sampling unit has a shape and a size suitable for the liquid sample in question. For example, the cavity may have the shape of a drop, a cone, a cylinder or e.g. an elongated cylinder. With such a shape, a small contact surface with the liquid may be obtained in relation to the cavity's volume. This may be advantageous, since a cylindrical sampling unit is easy to manufacture and arrange in different systems. The sampling unit may be detachably fitted in a system.

The sampling unit further comprises a blocking element, adapted to be in contact with the opening and the liquid in the system. The objective of the blocking element is to prevent a liquid flow in and out of the cavity when the pressure in the system is stable. At a stable pressure in the system, there may be minor pressure variations, which are substantially smaller than the variation between the resting and the operating pressures, e.g. in connection with disruptions in the system and changes in the air pressure. The blocking element is adapted, however, not to prevent the liquid flow at pressure variations from the resting pressure to the operating pressure or vice versa, since it may not resist flows caused by the compression and/or expansion of the liquid in connection with pressure variations. The blocking element thus allows the liquid to flow into the cavity when the pressure in the system increases from a resting pressure to an operating pressure, and out of the cavity when the pressure in the system drops back from the operating pressure to the resting pressure.

The blocking element may be adapted as a cover element, which at least partly covers the opening. The cover element may be a pressure-sensitive membrane, a perforated lid element or a fibre cloth. Pressure-sensitive membranes, lid elements and fibre cloths, e.g. filter paper, may prevent the mixing of liquids between the sampling unit's cavity and the system when the system has a stable pressure. Pressure-sensitive membranes may be made of a flexible material, e.g. a polymer material such as fluoropolymers. Pressure-sensitive membranes are easy to shape and may easily be adapted to the shape of the sampling unit. The element may also be shaped as a perforated lid made of e.g. aluminium or a polymer material. Such lids are also easy to manufacture and adapt for different purposes. The element may also be a fibre cloth, e.g. a fibre filter.

According to one example, the sampling unit's blocking element may be in the form of a buffer device, which may be arranged between the liquid in the system and the opening of the sampling unit. The buffer device may prevent the liquid's flow into or out of the cavity at a stable pressure, or at small pressure variations. Small pressure variations in the system may e.g. occur due to shakes or at temperature changes.

The buffer device is arranged between the liquid in the system and the sampling unit's cavity. The buffer device may comprise a channel or similar, which may be filled with liquid. When the pressure in the system increases, the liquid in the sampling unit comprising the buffer device is compressed, and when the pressure drops, the liquid in the sampling unit, including the buffer device, expands. The volume of the channel is arranged so that the volume corresponds to the volume change caused by the compression/expansion of the liquid at a certain pressure variation, which is smaller than the pressure variation between the resting pressure and the operating pressure. The exchange of liquid inside the cavity may thus be prevented at minor pressure variations, since at minor pressure variations, the liquid is compressed/expands less than the volume corresponding to the volume of the buffer device. If the pressure variation is equal to or greater than the pressure variation between the resting pressure and the operating pressure, the liquid is compressed/expands by a volume greater than the volume of the buffer device. The liquid's compression volume/expansion volume will thus reach the liquid in the cavity and an exchange of the liquids between the system and the cavity may occur. There is no remixing of the liquid in the buffer device, and when the sampling is complete the buffer device will be removed from the sampling unit. By way of the buffer device, small, frequent exchanges and mixtures of the liquid in the cavity may be prevented.

The buffer device's channel may be adapted, e.g. as a buffer pipe with a specific volume. The buffer pipe may also be used to reduce the exchange in a sampling unit at a given pressure variation. For example, if the exchange is optimal for a pressure variation of 12 bar and the system has a 15-bar pressure variation, the buffer device's volume may be determined to a compression volume corresponding to compression at an over pressure of 3 bar. When the pressure increases with up to 3 bar, the liquid is compressed to a volume corresponding to the volume in the buffer pipe, and the liquid in the cavity is therefore not impacted. At greater pressure variations, the liquid in the cavity also is compressed/expands.

According to another example, the blocking element may also be shaped as a check valve arrangement. The check valve arrangement is adapted to be in contact with the opening and the liquid in the system. The check valve arrangement comprises a first check valve, allowing the liquid to flow into the cavity and a second check valve, allowing the liquid to flow out of the cavity in connection with pressure variations. Each one of the check valves preferably has a specified activation pressure, which means that the valve is opened when the flow caused by the pressure variation has a higher pressure than the activation pressure of the check valve. In this manner, it is possible to prevent frequent exchange of the liquid inside the cavity at minor pressure variations in the system.

The sampling unit may comprise a flexible wall section, entailing that the cavity's volume increases when the sampling unit or the liquid is subjected to an over pressure, and that the cavity's volume decreases when the pressure drops after the liquid has been subjected to the over pressure. The sampling unit's volume may be increased as the flexible wall section springs out due to the pressure in the liquid flowing into the cavity, when the pressure in the system increases. When the pressure subsequently drops, the liquid inside the cavity expands and a small volume of the liquid flows out of the cavity, and the volume of the cavity is then reduced as well, as the flexible wall section springs back to its original shape. A sampling device with a flexible wall section is particularly suitable for use in systems where the pressure variation between the resting pressure and the operating pressure is not so great, e.g. in a fuel system's low pressure system, where the pressure variation is below approximately 12 bar.

A sampling unit with the flexible wall section may be used e.g. in combination with a cover element or a buffer device.

The invention uses a system which is subjected to pressure variations between a resting pressure and an operating pressure. The system comprises a hollow component, which contains or transports a liquid. The system comprises a sampling unit as described above, fitted into the hollow component. When the sampling unit is fitted into the component, which may occur e.g. through the component's wall, it comes into contact with the liquid inside a hollow part of the component.

The fitting of the sampling unit may occur in different ways. For example, the wall may be drilled, so that a through-hole is formed in the wall. Subsequently, the sampling unit is fitted and fastened in the holes with a suitable attaching device. For example, the sampling unit may be shaped as a screw with a hollow part constituting the cavity, and the attachment may be carried out with the help of threads on the outside of the screw. The sampling unit may, however, be fitted into the component with the help of other attachment methods, and may e.g. be placed inside a component and screwed onto the inside of the component wall, so that the liquid may flow into the cavity through the opening when the pressure increases, and out of the cavity when the pressure falls.

The sampling unit is preferably detachably fitted in the system. In this manner, the liquid may easily be emptied or removed from the cavity.

Preferably, the system is a fuel system comprising several components. The components consist of at least one fuel conduit, a feeding pump, a main fuel filter, a high pressure pump, an accumulator and an injection system, which components are connected with one or several fuel conduits. The high pressure pump, the accumulator and the injection system constitute components in the fuel system's high pressure system, and the feeding pump and the main fuel filter constitute components in the fuel system's low pressure system. The pressure in the high pressure system may be approximately 1,800-2,500 bar, and the pressure in the low pressure system may be approximately 6-15 bar. At least one sampling unit as described above may be fitted in at least one of the fuel system's components.

The sampling unit may be fitted into a component in the fuel system's low pressure system. It is easy to replace and monitor the sampling unit, if it is fitted into the fuel system's low pressure system. When the sampling device is fitted into the low pressure system, it preferably comprises the above mentioned flexible wall section, which entails that the cavity's volume increases when the pressure in the system increases from a resting pressure to an operating pressure, and that the cavity's volume decreases when the pressure in the system drops from the operating pressure to the resting pressure. Alternatively, the sampling unit may comprise the buffer device mentioned above, and in this manner prevent frequent exchange of the liquid inside the cavity at small pressure variations. The sampling unit may e.g. be fitted into the main fuel filter.

The sampling unit may also be fitted into a component in the fuel system's high pressure system. When the sampling unit is fitted into the high pressure system, the sampling is only impacted a little, or not at all, by temperature variations in the surrounding environment, since the pressure in the high pressure system far exceeds the ambient pressure. Since the pressure in the high pressure system is high, often over 2,000 bar, a greater compression of the liquid is achieved inside the cavity, than what is the case in the low pressure system. When the sampling unit is fitted into the high pressure system, the sampling unit may comprise the buffer device described above, and in this manner prevent too large an exchange of liquid inside the cavity. When the buffer device is adapted as a pipe, its volume may be determined as a compression volume corresponding to compression at a specific pressure variation.

Preferably, the liquid is a fuel, the system is a fuel system, and the substance to be analysed is sulphur.

In order to determine the sulphur level of the fuel, the liquid must be analysed. This suitably occurs after the sampling unit has been removed from the fuel system. The sulphur level of the fuel may be analysed according to standard methods, such as those described in Swedish Standard SS-EN ISO 20884 (Petroleum Products - Determination of Sulphur Content in Fuels - Wavelength-dispersive X-ray Spectroscopy (ISO 20884:2011) and/or Swedish Standard SS-EN ISO 20846 (Petroleum Products - Determination of Sulphur Content in Fuels - Ultraviolet Fluorescence Method (ISO 20846:2011). From the analysis result an indication is obtained, as to whether the tested fuel has a sulphur level that is higher than the recommended level for the fuel, which is below 10 ppm.

Other advantages of the invention are set out in the description below with reference to the enclosed drawings.

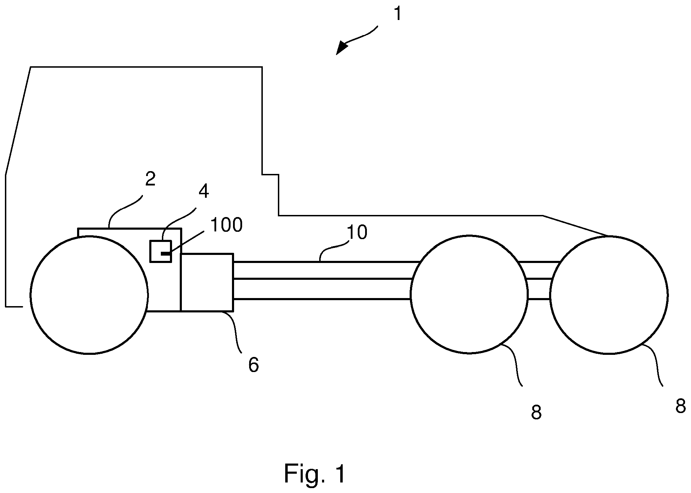

Fig. 1 shows a schematic side view of a vehicle 1, which vehicle comprises a fuel system 4 for a combustion engine 2, which system comprises a sampling unit 100.

The combustion engine 2 is connected to a gearbox 6, which is further connected to the driving wheels 8 of the vehicle 1 via a transmission. The vehicle also comprises a chassis 10.

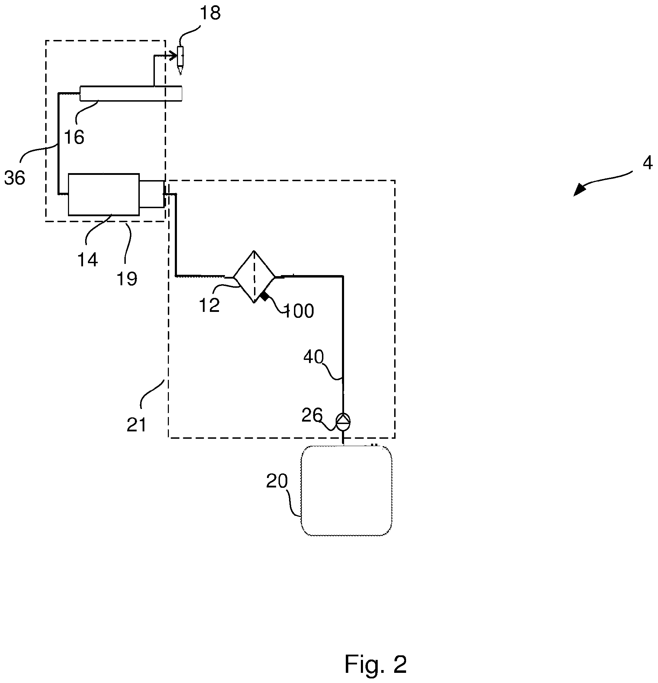

Fig. 2 shows an example of a coupling diagram for a fuel system 4 for a combustion engine 2. The sampling unit 100 may, according to the present invention, be used in e.g. such a fuel system, but other fuel system versions may be relevant. The sampling unit may also be used in other liquid systems, e.g. within the process industry.

The fuel system 4 comprises several components, including a main fuel filter 12, a high pressure pump 14, an accumulator in the form of a so-called common rail 16, and an injection system 18, schematically displayed in the form of fuel injectors arranged in the combustion engine 2 (the combustion engine 2 is displayed in Fig. 1). Alternatively, the common rail 16 may be replaced by another form of an injection system 18, e.g. a piezo or a unit injection system. The high pressure pump 14, the common rail 16 and the injection system 18 constitute components in the fuel system's 4 high pressure system 19. A sampling unit may, according to the present invention, be placed in any of the high pressure system's components, e.g. in a fuel conduit between the high pressure pump 14 and the common rail 16.

The fuel system 4 also comprises a fuel tank 20 and a feeding pump 26. These components may be arranged at the vehicle's chassis 10 (chassis 10 is displayed in Fig. 1). The main fuel filter 12 is arranged downstream of the pump 26 and upstream of the high pressure pump 14 in the fuel system 4. The sampling unit 100 may be fitted into the main fuel filter 12, as displayed in this example, but other placements are also possible, e.g. in a fuel conduit 40.

The feeding pump 26 pressurises the fuel in a low pressure system 21 of the fuel system, and feeds the fuel from the fuel tank 20, via the fuel conduit 40, through the main fuel filter 12, and further along to the high pressure pump 14. The fuel is then fed, at a high pressure, to the common rail 16 and further along to the injection system 18.

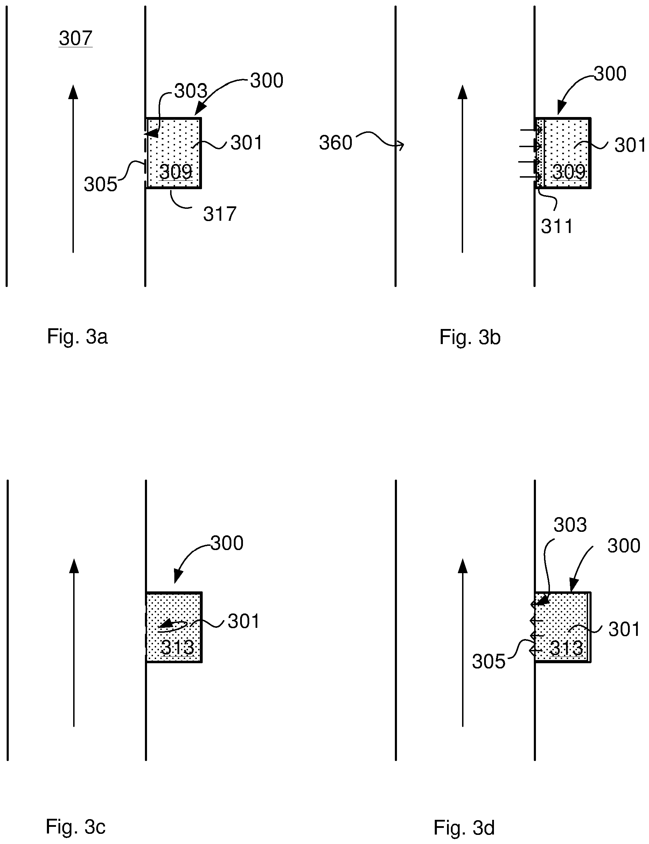

Figs. 3a-3d schematically show the function of a sampling unit 300. The sampling unit 300 comprises a wall section 317, which partly surrounds a cavity 301, an opening 303 and a blocking element 305. The blocking element 305 and the opening 303 are arranged to be in contact with a liquid 307, e.g. a fuel, to be analysed. During a time period, the liquid sample may be collected continuously from the liquid 307 in the system, with which the sampling unit 300 is in contact. The sampling is done by way of the cavity first being filled with a starting liquid, which may be a "pure" liquid 309, e.g. fuel whose sulphur level is equal to or below 10 ppm, as displayed in Fig. 3a. Fig. 3b shows that during an over pressure the liquid 309, which is in the cavity 301, is compressed during an over pressure, and therefore a small amount of liquid 311 from a surrounding component 360, which in this case is a fuel conduit, may flow through the opening 303, and through the blocking element 305. Fig. 3c shows that a mixing of the liquids 311 and 309 occurs during an over pressure, shown in Fig. 3b, and a liquid 313 with a new composition is formed. Fig. 3d shows that the liquid 313 in the cavity 301 expands after the pressure drops, and approximately a similar amount of liquid 313 that was let in (liquid 311) flows out through the opening 303 and the blocking element 305. During the continuous sampling period there are several cycles of over pressure and subsequent pressure drops.

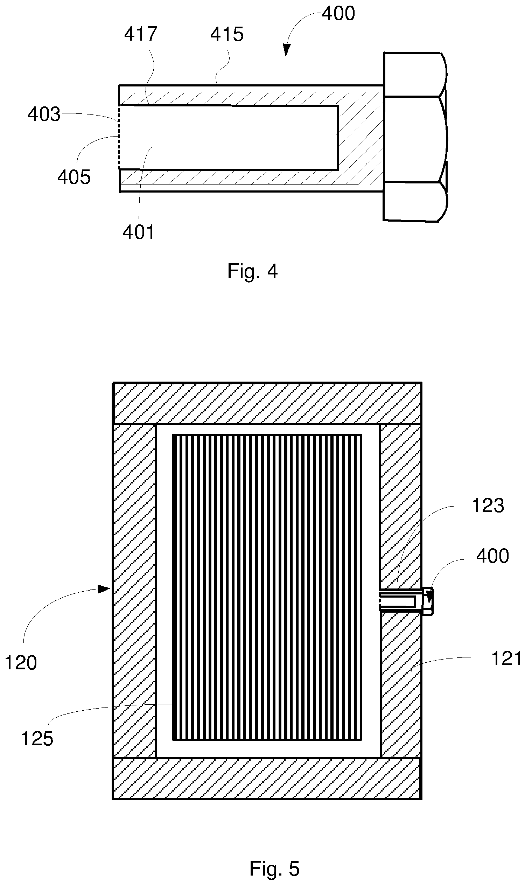

Fig. 4 shows an example of a possible design of the sampling unit. The sampling unit 400 comprises a wall section 417, which partly surrounds a cavity 401, an opening 403 and a blocking element 405. The sampling unit 400 is shaped like a screw, i.e. a screw comprising a hollow part constituting the cavity, and the attachment is carried out with the help of threads 415 on the outside of the screw. The cavity 401 has an elongated cylindrical shape. The blocking element 405 may e.g. be a pressure-sensitive membrane of a polymer material.

Fig. 5 shows that the sampling unit 400 is fitted into a main fuel filter 120, which constitutes a component in a low pressure system 21 in a fuel system 4 (displayed in Fig. 2) and comprises a filter 125. The sampling unit 400 is screwed into an opening 123 in one of the walls 121 of the main fuel filter.

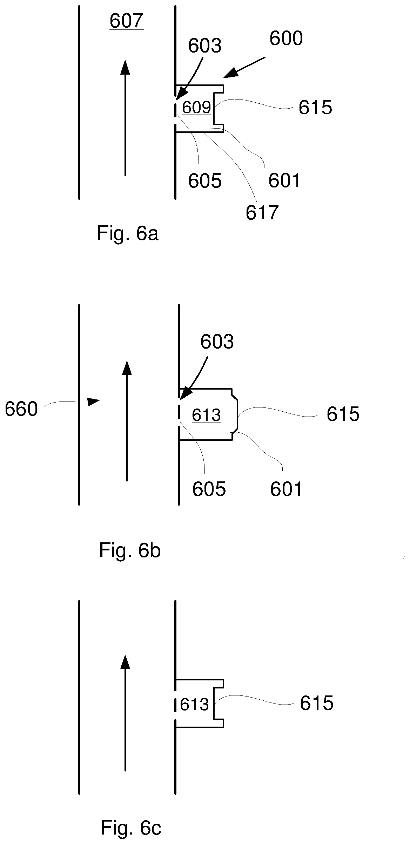

Figs. 6a-6c schematically show the function of a sampling unit 600, which comprises a wall section 617 and a flexible wall section 615, which together partly surround a cavity 601. Similarly, as described above in connection with Figs. 3a-3d, the sampling unit 600 comprises the cavity 601, an opening 603 and a blocking element 605. The opening 603 and the blocking element 605 are arranged to be in contact with a liquid 607, e.g. a fuel, which is to be analysed. The liquid sample may be collected continuously from the liquid 607, with which the sampling unit 600 is in contact. The sampling is done by way of the cavity first being filled with a starting liquid, which may be a "pure" liquid 609, e.g. fuel whose sulphur level is below 10 ppm, as displayed in Fig. 6a. During over pressure the flexible wall section 615 springs out, and thus a larger volume is provided in the cavity 601, as displayed in Fig. 6b. A small amount of liquid from a surrounding component 660, which in this case is a fuel conduit, may flow in through the opening 603 and the blocking element 605 because of the compression, mix with the liquid 609 and form a mixed liquid 613. Fig. 6c shows that when the pressure drops back to the resting pressure, which may be equal to the atmospheric pressure, the flexible wall section 615 springs back to its original shape, and approximately the same amount of liquid as the amount let in flows out through the opening 603 and the blocking element 605. The flexible wall section 615 entails that the volume of the cavity 601 increases during over pressure, and that the volume of the cavity 601 volume decreases when the pressure drops back to the resting pressure. In this way, a volume of liquid 607 from the component 660 may be mixed with the liquid 609 in the cavity.

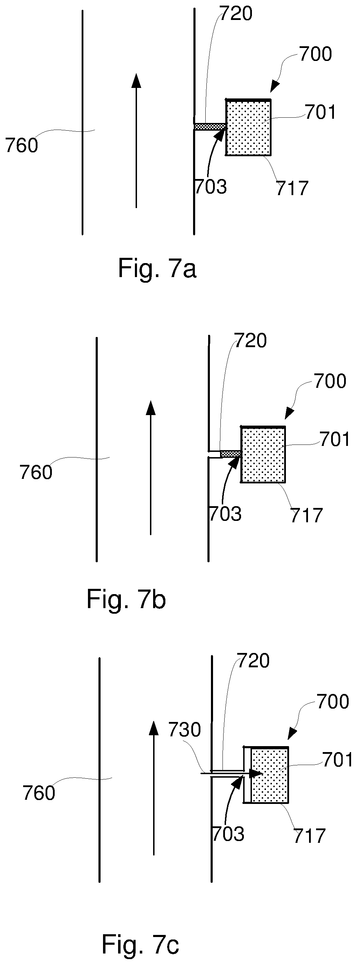

Fig. 7a shows another sampling unit 700. The sampling unit 700 comprises a wall section 717, which partly surrounds a cavity 701, an opening 703 and a blocking element in the form of a buffer pipe 720. The cavity 701 has a cylindrical shape. The buffer pipe 720 is arranged between a hollow component 760 and the opening 703 of the sampling unit 700. The pipe 720 constitutes a channel and is filled with liquid.

Fig. 7b shows that, at a small pressure increase the liquid is compressed with a small volume, corresponding to a part of the buffer pipe's volume. The buffer pipe's volume is arranged so that the pipe may receive a small part of liquid when only a small pressure variation in the liquid occurs in relation to the ambient pressure, e.g. because of disruptions in the system, variations in the ambient air pressure or temperature changes, which allow for minor pressure variations.

Fig. 7c shows a situation where the pressure increases from a resting pressure to an operating pressure. The liquid is compressed with a volume that is greater than the volume in the pipe 720, and therefore a part of the liquid from the hollow component 760 may flow into the cavity 701, as illustrated with the help of the arrow 730. When the pressure subsequently drops back from the operating pressure to the resting pressure, the liquid expands, e.g. by a volume which is greater than the volume of the pipe 720 (not displayed), and as a result the cavity and the pipe 720 are filled with the liquid from the cavity. During the sampling period there are several cycles of compression and expansion, and some of the liquid may therefore be replaced in the cavity 701. In this way, it is possible to ensure that the liquid in the cavity is mixed with the liquid outside the cavity at operating conditions, where the pressure variation occurs between the resting pressure and the operating pressure.

The foregoing description of the preferred embodiments of the present invention has been furnished with the objective of illustrating and describing the invention. The embodiments described are not intended to be exhaustive, or to limit the invention.

## Claims

1. Method for analysis of a liquid with a sampling unit (100; 300; 400; 600; 700), wherein the sampling unit (100; 300; 400; 600; 700) is adapted to be fitted into a system which contains or transports a liquid, and wherein the sampling unit (100; 300; 400; 600; 700) comprises a wall section (317; 417; 617; 717) which partly surrounds a cavity (301; 401; 601; 701) adapted to be filled with liquid, an opening (303; 403; 603; 703) equipped with a blocking element (305; 405; 605; 705; 720), which is adapted to be in contact with the liquid in the system, which blocking element (305; 405; 605; 705; 720) prevents the liquid from flowing in or out of the cavity at a substantially stable pressure in the system, but allows the liquid to flow into the cavity (301; 401; 601; 701) when the pressure in the system increases from a resting pressure to an operating pressure, and out of the cavity when the pressure in the system drops back from the operating pressure to the resting pressure,
wherein the method comprises the steps of
a) filling the sampling unit (100; 300; 400; 600; 700) with a starting liquid;
b) placing or fitting the sampling unit (100; 300; 400; 600; 700) into a system (4, 19, 21) which contains or transports a liquid and in which the liquid is subjected to pressure variations between a resting pressure and an operating pressure, which is higher than the resting pressure;
c) increasing the pressure from the resting pressure to the operating pressure to cause a flow of the liquid in the system (4, 19, 21) into the sampling unit (100; 300; 400; 600; 700);
d) reducing the pressure from the operating pressure to the resting pressure to cause a flow of liquid out of the sampling unit (100; 300; 400; 600; 700);
e)removing the sampling unit (100; 300; 400; 600; 700) from the system (4, 19, 21) after a time period;
f) removing the liquid accumulated in the sampling unit (100; 300; 400; 600; 700), and
g) analysing the level of a substance in the liquid with an analysis method adapted to the substance to be analysed.

2. Method according to claim 1, **characterised in that** the liquid is a fuel and the system is a fuel system (4) and the substance to be analysed is sulphur.

## Patentansprüche

1. Verfahren zur Analyse einer Flüssigkeit mit einer Probenahmeeinheit (100; 300; 400; 600; 700), wobei die Probenahmeeinheit (100; 300; 400; 600; 700) dazu eingerichtet ist, in ein System eingesetzt zu werden, das eine Flüssigkeit enthält oder transportiert, und wobei die Probenahmeeinheit (100; 300; 400; 600; 700) einen Wandabschnitt (317; 417; 617; 717), der eine zum Befüllen mit Flüssigkeit eingerichtete Vertiefung (301; 401; 601; 701) teilweise umgibt, und eine Öffnung (303; 403; 603; 703) aufweist, die mit einem Absperrelement (305; 405; 605; 705; 720) versehen ist, welches dazu ausgeführt ist, in Kontakt mit der Flüssigkeit in dem System zu stehen, wobei das Absperrelement (305; 405; 605; 705; 720) die Flüssigkeit daran hindert, bei einem im Wesentlichen stabilen Druck in dem System in die Vertiefung oder aus ihr heraus zu fließen, es der Flüssigkeit jedoch erlaubt, in die Vertiefung (301; 401; 601; 701) zu fließen, wenn der Druck in dem System von einem Ruhedruck auf einen Betriebsdruck steigt, und aus der Vertiefung heraus, wenn der Druck in dem System von dem Betriebsdruck zurück auf den Ruhedruck fällt,
wobei das Verfahren die Schritte umfasst:
a) Füllen der Probenahmeeinheit (100; 300; 400; 600; 700) mit einer Startflüssigkeit,
b) Platzieren oder Einsetzen der Probenahmeeinheit (100; 300; 400; 600; 700) in ein System (4, 19, 21), welches eine Flüssigkeit enthält oder transportiert und in dem die Flüssigkeit Druckänderungen zwischen einem Ruhedruck und einem Betriebsdruck ausgesetzt wird, der höher ist als der Ruhedruck,
c) Erhöhen des Drucks von dem Ruhedruck auf den Betriebsdruck, um einen Strom der Flüssigkeit in dem System (4, 19, 21) in die Probenahmeeinheit (100; 300; 400; 600; 700) zu verursachen,
d) Verringern des Drucks von dem Betriebsdruck auf den Ruhedruck, um einen Flüssigkeitsstrom aus der Probenahmeeinheit (100; 300; 400; 600; 700) zu bewirken,
e) Entnehmen der Probenahmeeinheit (100; 300; 400; 600; 700) aus dem System (4, 19, 21) nach einer Zeitdauer,
f) Entnehmen der in der Probenahmeeinheit (100; 300; 400; 600; 700) angesammelten Flüssigkeit, und
g) Analysieren des Gehalts einer Substanz in der Flüssigkeit mit einem auf die zu analysierende Substanz abgestimmten Analyseverfahren.

2. Verfahren nach Anspruch 1, **dadurch gekennzeichnet, dass** die Flüssigkeit ein Kraftstoff ist und das System ein Kraftstoffversorgungssystem (4) ist und die zu analysierende Substanz Schwefel ist.

## Revendications

1. Procédé d'analyse d'un liquide avec une unité d'échantillonnage (100 ; 300 ; 400 ; 600 ; 700), dans lequel l'unité d'échantillonnage (100 ; 300 ; 400 ; 600 ; 700) est adaptée pour être ajustée dans un système qui contient ou transporte un liquide, et dans lequel l'unité d'échantillonnage (100 ; 300 ; 400 ; 600 ; 700) comprend une section de paroi (317 ; 417 ; 617 ; 717) qui entoure partiellement une cavité (301 ; 401 ; 601 ; 701) adaptée pour être remplie avec du liquide, une ouverture (303 ; 403 ; 603 ; 703) équipée d'un élément de blocage (305 ; 405 ; 605 ; 705 ; 720), qui est adapté pour être en contact avec le liquide dans le système, lequel élément de blocage (305 ; 405 ; 605 ; 705 ; 720) empêche le liquide de s'écouler dans ou hors de la cavité à une pression sensiblement stable dans le système, mais permet au liquide de s'écouler dans la cavité (301 ; 401 ; 601 ; 701) lorsque la pression dans le système augmente d'une pression de repos à une pression de fonctionnement, et hors de la cavité lorsque la pression dans le système redescend de la pression de fonctionnement à la pression de repos,
dans lequel le procédé comprend les étapes :
a) du remplissage de l'unité d'échantillonnage (100 ; 300 ; 400 ; 600 ; 700) avec un liquide de démarrage ;
b) du placement ou de l'ajustement de l'unité d'échantillonnage (100 ; 300 ; 400 ; 600 ; 700) dans un système (4, 19, 21) qui contient ou transporte un liquide et dans lequel le liquide est soumis à des variations de pression entre une pression de repos et une pression de fonctionnement, qui est supérieure à la pression de repos ;
c) de l'augmentation de la pression à partir de la pression de repos jusqu'à la pression de fonctionnement pour provoquer un flux du liquide dans le système (4, 19, 21) dans l'unité d'échantillonnage (100 ; 300 ; 400 ; 600 ; 700) ;
d) de la réduction de la pression à partir de la pression de fonctionnement jusqu'à la pression de repos pour provoquer un flux de liquide hors de l'unité d'échantillonnage (100 ; 300 ; 400 ; 600 ; 700) ;
e) du retrait de l'unité d'échantillonnage (100 ; 300 ; 400 ; 600 ; 700) du système (4, 19, 21) après une période de temps ;
f) du retrait du liquide accumulé dans l'unité d'échantillonnage (100 ; 300 ; 400 ; 600 ; 700), et
g) de l'analyse du niveau d'une substance dans le liquide avec un procédé d'analyse adapté à la substance à analyser.

2. Procédé selon la revendication 1, **caractérisé en ce que** le liquide est un carburant et le système est un système de carburant (4) et la substance à analyser est du soufre.
